# EUROPÄISCHE PATENTSCHRIFT

(11) **EP 2 880 022 B1**
(45) Veröffentlichungstag und Bekanntmachung des Hinweises auf die Patenterteilung: **08.06.2016**
(21) Anmeldenummer: 13741788.7
(22) Anmeldetag: 29.07.2013
(51) Int. Cl.: C07D 307/68

(54) **MEHRSTUFIGES VERFAHREN ZUR HERSTELLUNG VON ALKALIMETALLSALZEN SPEZIELLER 4-HYDROXY-2-OXO-2,5-DIHYDROFURAN-3-CARBONSÄUREESTER**
MULTI-STAGE PROCEDURE FOR PRODUCING ALKALI METAL SALTS OF SPECIAL 4-HYDROXY-2-OXO-2,5- DIHYDROFURAN-3-CARBOXYLIC ACID ESTERS
PROCÉDÉ EN PLUSIEURS ÉTAPES DESTINÉ À LA FABRICATION DE SELS DE MÉTAL ALCALIN À BASE D'ESTERS D'ACIDE 4-HYDROXY-2-OXO-2,5-DIHYDROFURANE-3-CARBONIQUE

(30) Priorität: 01.08.2012 EP 12178859
(43) Veröffentlichungstag der Anmeldung: 10.06.2015
(73) Patentinhaber: Bayer CropScience AG, 40789 Monheim am Rhein (DE)
(72) Erfinder: MORADI, Wahed, Ahmed, 40789 Monheim (DE); FUNKE, Christian, 42799 Leichlingen (DE); FARIDA, Taraneh, 50259 Pulheim (DE); SCHNATTERER, Albert, 51373 Leverkusen (DE); ROSELLEN, Reiner, 51373 Leverkusen (DE); BREHME, Volker, 48301 Nottuln (DE); RINKER, Stefanie, 46569 Hünxe (DE)
(74) Vertreter: BIP Patents
(86) Internationale Anmeldenummer: PCT/EP2013/065904
(87) Internationale Veröffentlichungsnummer: WO 2014/019982

(56) Entgegenhaltungen:
- WO-A1-2009/036899
- WO-A1-2012/117015

## Beschreibung

Die vorliegende Erfindung betrifft ein mehrstufiges Verfahren zur Herstellung von Alkalimetallsalzen spezieller 4-Hydroxy-2-oxo-2,5-dihydrofuran-3-carbonsäureester, das vom Malonsäureester ausgeht und das ohne Isolierung von Zwischenstufen auskommt.

Die Herstellung von Hydroxy-2-oxo-2,5-dihydrofuran-3-carbonsäureester, der entsprechenden Tautomere, bzw. von deren Alkalimetallsalzen und auch deren Verwendung als Baustein in der Synthese biologisch aktiver Verbindungen ist bekannt (WO 2011/018180, WO 2009/036899, J. Chem. Soc., Perkin Trans. 1, 1985, Seiten 1567 bis 1576, Berichte der Deutschen Chemischen Gesellschaft (1911), 44, 1759 - 1765). Die bekannten Verfahren haben jedoch, wie nachfolgend beschrieben, Nachteile.

WO-A-2012/117015 offenbart ein Verfahren zur Herstellung von Natrium- oder Kaliumsalzen der 4-Hydoxy-2-oxo-2,5-dihydrofuran-3-carbonsäureester. Das Verfahren wird gemäß WO-A-2012/117015 aber nicht in Anwesenheit eines Phasentransferkatalysators durchgeführt. Darüber hinaus verwendet das Verfahren im Schritt (ii) die teure Komponente Chloressigsäureethylester zum Azeotropieren der wässrigen Phase. Die partielle Hydrolyse des Chloressigsäureethylesters führt zu hohen Edukt- und Abfallkosten.

WO 2011/018180 beschreibt eine Herstellung von Hydroxy-2-oxo-2,5-dihydrofuran-3-carbonsäureester ausgehend von Malonsäureester. Dieser wird mit einer Halogenacetylchloridverbindung in Gegenwart einer Base umgesetzt (siehe Reaktionsschema 1). Nach Zugabe von Wasser wird der gewünschte 4-Hydroxy-2-oxo-2,5-dihydrofuran-3-carbonsäureester erhalten. Die Base ist so gewählt, dass sie den Malonsäureester zu deprotonieren vermag, wodurch das Enolat des Malonsäureesters entsteht, das dann durch die Halogenacetylchloridverbindung acetyliert wird. Geeignete Basen sind insbesondere Alkoholate der allgemeinen Formel M(OR^{a})_{b}, in denen M für Na⁺, K⁺, Mg²⁺, b für 1 oder 2 und R^{a} für Methyl oder Ethyl steht. Als bevorzugt wird Natriummethylat genannt. Nach erfolgtem Ringschluss wird das gewünschte Produkt zusammen mit einem anorganischen Salz, das als Nebenprodukt anfällt, erhalten (z.B. NaCl, falls ein Natriumalkoholat als Base eingesetzt wird).

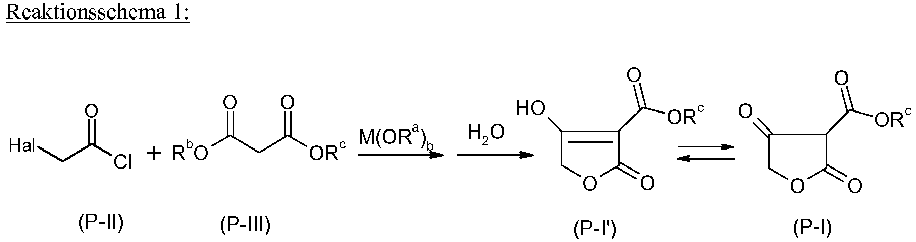

Die Abtrennung des anorganischen Salzes von der Reaktionsmischung, vor allem dann, wenn es sich um NaCl handelt, ist, wenn überhaupt, nur durch einen sehr großen technischen Aufwand zu erreichen, da die Verbindungen der Formeln (P-I') und (P-I) sehr gut wasserlöslich sind. Eine Destillation ist nicht möglich, da sich die Verbindungen der Formeln (P-I') und (P-I) bei höheren Temperaturen unter Freiwerden von CO₂ zersetzen. Das anorganische Salz wird deshalb nicht abgetrennt. Stattdessen wird es in die nachfolgende Reaktion mit eingebracht und kann erst nach erfolgter Weiterreaktion der Verbindungen der Formeln (P-I') und/oder (P-I) abgetrennt werden.

WO 2009/036899 beschreibt eine Synthese von Salzen von 4-Hydroxy-2-oxo-2,5-dihydrofuran-3-carbonsäureester, die von einem Malonsäureester-Kaliumsalz ausgeht und in der unter Verwendung von Chloressigsäureester und einer Alkoholatbase, z.B. Natriummethanolat, das entsprechende Salz eines 4-Hydroxy-2-oxo-2,5-dihydrofuran-3-carbonsäureesters hergestellt wird (siehe Reaktionsschema 2). Bei dieser Reaktion entstehen keine anorganischen Salze, die mitgeschleppt werden müssen. Allerdings ist es zu deren Abtrennung notwendig, das im Schritt 1 verwendete polare Lösungsmittel Dimethylformamid (DMF) bzw. Dimethylacetamid (DMA) als Lösungsmittel vollständig zu entfernen. DMF und DMA sind zum einen reproduktionstoxisch, zum anderen teuer und die Entfernung und Recyclierung sehr aufwändig. Ein weiterer Nachteil ist der Einsatz des teuren Monokaliumsalzes des Malonsäureesters.

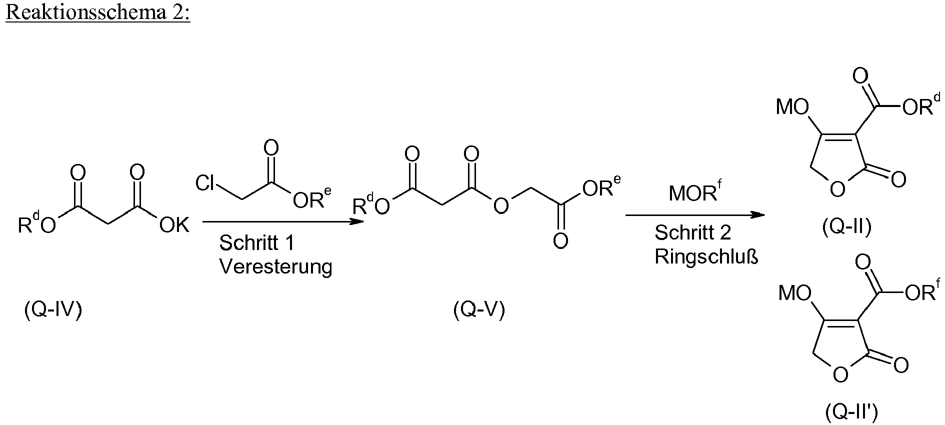

Schritt 1 der vorgenannten Reaktion stellt eine Veresterung dar, in der als Nebenprodukt Kaliumchlorid anfällt, das nachfolgend von der Reaktionsmischung über eine Wasserwäsche abgetrennt wird. In Schritt 2 findet der Ringschluss zur gewünschten Verbindung (Q-II) statt, wobei es zu einer Umesterung kommen kann, weshalb das Produkt als ein Gemisch aus den Verbindungen der Formeln (Q-II) und (Q-II') anfällt. Verbindungen der Formel (Q-IV) sind Feststoffe, kommerziell erhältlich oder können nach bekannten Verfahren hergestellt werden (vgl. J. Am. Chem. Soc. 1944, Nr. 66, Seite 1286, EP-A-950653, WO 2008/150487).

Die Herstellung des Produktes nach dem in WO 2009/036899 genannten Verfahren ist technisch unvorteilhaft, da das Verfahren von teuren, als Feststoff vorliegenden Malonsäureester-Kaliumsalzen der Formel (Q-IV) ausgeht. Bei einer technischen Herstellung ist das Verwenden von Feststoffen als Ausgangsmaterialien grundsätzlich unerwünscht, da die technische Handhabung von Feststoffen schwierig ist und es öfter zu einem Lösungsmittelwechsel kommt, was insgesamt zu einem erheblichen technischen Aufwand bei der Reaktionsdurchführung führt.

Ausgehend von den bekannten Verfahren zur Herstellung von Salzen der 4-Hydroxy-2-oxo-2,5-dihydrofuran-3-carbonsäureester stellt sich nun die Aufgabe, wie diese einfach und kostengünstig hergestellt werden können, so dass das Verfahren auch zur technischen Herstellung der gewünschten Verbindung verwendet werden kann.

Wünschenswert wäre insbesondere ein Verfahren zu finden, das ausgehend von einfachen Startmaterialien ohne aufwändige Isolierung der Zwischenstufen und dem Wechseln von Lösungsmitteln auskommt. Unter einfachen, kostengünstigen Verfahren werden solche Verfahren verstanden, die ohne großen finanziellen Aufwand durchzuführen sind, weil die Ausgangsstoffe beispielsweise kostengünstig und/oder keine karzinogene, mutagene oder reprotoxische Aktivität (CMR-Aktivität) besitzen, das Verfahren mit wenigen Verfahrensschritten auskommt bzw. sogar als "Eintopfreaktion" (d.h. ohne dass eine Isolierung von Zwischenstufen notwendig ist) durchzuführen ist, und/oder das gewünschte Natrium- oder Kaliumsalz der 4-Hydroxy-2-oxo-2,5-dihydrofuran-3-carbonsäureester in einer ausreichend hohen Ausbeute und Reinheit erhalten wird. Ferner ist es von Vorteil, ein Verfahren bereitzustellen, das Ressourcen schont, in dem es z.B. weniger Energie verbraucht und/oder selektiv ist, d.h. dass nur im geringen Maße Nebenprodukte entstehen.

Es wurde nun ein Verfahren zur Herstellung von Alkalimetallsalzen, insbesondere Natrium- oder Kaliumsalzen, spezieller 4-Hydroxy-2-oxo-2,5-dihydrofuran-3-carbonsäureester der allgemeinen Formel (I) gefunden, das ohne aufwändige Isolierung und/oder Aufreinigung von Zwischenstufen auskommt, und in dem das Lösungsmittel nicht gewechselt werden muss. Ferner wird, entgegen dem Stand der Technik, kein reproduktionstoxisches Lösungsmittel benötigt. Das erfindungsgemäße Verfahren ist somit besonders einfach, ressourcen-schonend und kostengünstig durchführbar. Gleichfalls sind die verwendeten Ausgangsmaterialien preiswert. Das Verfahren kann sowohl kontinuierlich als auch diskontinuierlich ablaufen.

Es ist bekannt, dass Hydroxy-2-oxo-2,5-dihydrofuran-3-carbonsäureester auch als Tautomere vorliegen können, nämlich als 2,4-Dioxotetrahydrofuran-3-carbonsäureester. Jede Bezugnahme hier auf Hydroxy-2-oxo-2,5-dihydrofuran-3-carbonsäureester umfasst demzufolge auch das entsprechende Tautomer.

Gegenstand der Erfindung ist somit das unten beschriebene Verfahren zur Herstellung eines Alkalimetallsalzes eines 4-Hydroxy-2-oxo-2,5-dihydrofuran-3-carbonsäureesters der Formel (I) 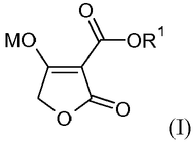 worin

M für ein Alkalimetall, vorzugsweise Natrium oder Kalium, steht und der Rest R¹ die unten angegebene Bedeutung hat, wobei das Alkalimetallsalz des 4-Hydroxy-2-oxo-2,5-dihydrofuran-3-carbonsäureesters der Formel (I), wie bereits beschrieben, in den folgenden tautomeren Formen vorliegen kann:

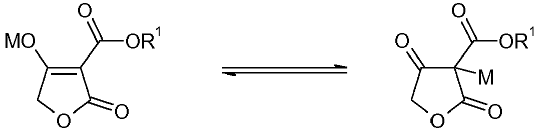

Die Anmeldung betrifft demzufolge ein Verfahren zur Herstellung eines Alkalimetallsalzes, insbesondere Natrium- oder Kaliumsalzes, eines 4-Hydroxy-2-oxo-2,5-dihydrofuran-3-carbonsäureesters der Formel (I), das die folgenden Schritte umfasst:
Schritt (i): Umsetzen eines Malonsäureesters der allgemeinen Formel (II) 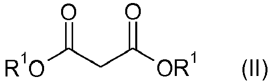 mit einer alkoholischen (vorzugsweise methanolischen oder ethanolischen) ggf. wasserenthaltenden Alkalimetallhydroxidlösung, insbesondere Natrium- oder Kaliumhydroxidlösung zum entsprechenden Malonsäureester-Mono-Alkalimetallsalz der Formel (III) 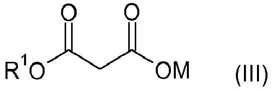 ggf. bevorzugt in Anwesenheit einer aromatischen Kohlenwasserstoffverbindung (aromatischer Kohlenwasserstoff) als Lösungsmittel (Variante [i-B]) oder in Substanz mit ggf. anschließender Zugabe der aromatischen Kohlenwasserstoffverbindung (Variante [i-A]) zur Reaktionsmischung und bevorzugtem Entfernen des im Reaktionsgemisch vorhandenen Alkohols und ggf. des Wassers, wobei das Malonsäureester-Mono-Alkalimetallsalz der Formel (III) nach Entfernen des Alkohols und ggf. des Wassers dann als Suspension in dem aromatischen Kohlenwasserstoff vorliegt, und wobei der von Beginn an vorhandene oder nach Umsetzung zugegebene aromatischen Kohlenwasserstoff als Schleppmittel zum Entfernen von Wasser dienen kann;
Schritt (ii): Umsetzen des Malonsäureester-Mono-Alkalimetallsalzes der Formel (III) aus Schritt (i) mit einem Chloressigsäureester der Formel (IV) 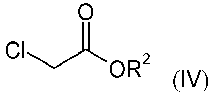 in Gegenwart mindestens eines Phasentransferkatalysators zu einer Verbindung der Formel (V) 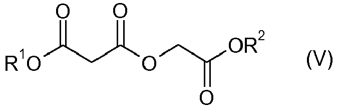 wobei der Phasentransferkatalysator ein organisches Ammonium- oder Phosphoniumsalz ist,
Schritt (iii): Umsetzen der Verbindung der Formel (V) durch Zugabe eines Alkalimetallalkoholats der allgemeinen Formel (VI)

   M'OR³ (VI)

   in einer Ringschlussreaktion, wodurch das Alkalimetallsalz des 4-Hydroxy-2-oxo-2,5-dihydrofuran-3-carbonsäureesters der Formel (I) hergestellt wird 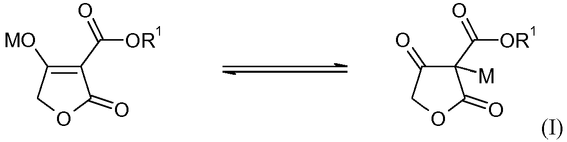 wobei in den vorgenannten allgemeinen Formeln (I) bis (VI)
   R¹, R² und R³ jeweils unabhängig voneinander für C₁-C₁₂-Alkyl stehen; bevorzugt stehen R¹, R² und R³ unabhängig voneinander für C₁-C₆-Alkyl (z.B. Methyl oder Ethyl); und
   M und M' unabhängig voneinander für ein Alkalimetall, vorzugsweise Natrium oder Kalium in der entsprechenden Oxidationsstufe steht.

In einer bevorzugten Ausführungsform der Erfindung sind die Reste (Substituenten) R¹, R² und R³ in den allgemeinen Formeln (I) bis (VI) gleich. In einer besonders bevorzugten Ausführungsform sind R¹, R² und R³ in den allgemeinen Formeln (I) bis (VI) gleich und stehen für Methyl oder Ethyl.

Üblicherweise stammt der Rest R¹ im Produkt der Formel (I) aus dem eingesetzten Ester der Formel (II). Darüber hinaus ist es auch möglich, dass der Substituent R¹ aus dem eingesetzten Chloressigester der Formel (IV), dem Alkoholat der Formel (VI) oder den bei der Umsetzung vorhandenen Alkoholen entstammt. Bei dem erhaltenen Produkt der Formel (I) kann es sich also in der Regel um eine Mischung aus mehreren Produkten handeln, die sich im Substituenten R¹ voneinander unterscheiden. Sind beispielsweise R¹, R² und R³ unterschiedlich, so können demnach drei Produkte der Formel (I) entstehen, mit jeweils R¹, R² oder R³ als entsprechendem Substituent.

Es ist erfindungsgemäß bevorzugt, dass R¹ und R² für den gleichen Rest (z.B. Methyl oder Ethyl) stehen. Sind R¹ und R² verschieden, so kann die Ringschlussreaktion in Schritt (iii) zu den Verbindungen der Formeln (I) und (I') führen (siehe Reaktionsschema 3).

Gleiches gilt auch für R³, auch R³ sollte bevorzugt gleich R¹ bzw. R² sein, das heißt insbesondere bevorzugt sind R¹, R² und R³ gleich.

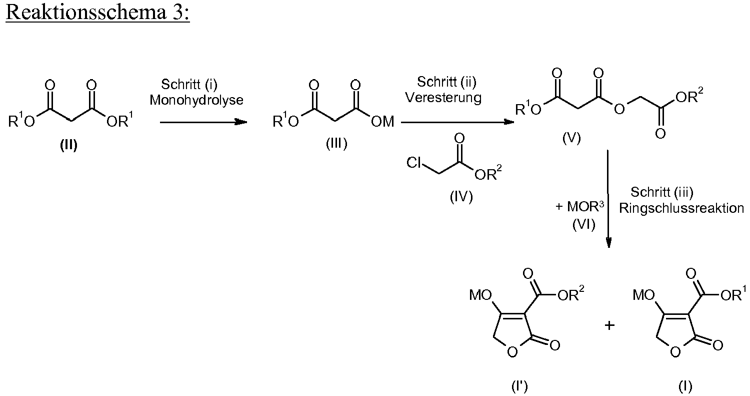

Unter alkoholischer Alkalimetallhydroxidlösung werden Lösungen verstanden, in denen Alkalimetallhydroxide, insbesondere Natrium- oder Kaliumhydroxid in einem oder mehreren Alkoholen, vorzugsweise Methanol und/oder Ethanol gelöst vorliegt. Diese Lösungen können Wasser enthalten.

Erstaunlicherweise wurde gefunden, dass für die Herstellung der alkoholischen Alkalimetallhydroxidlösung sogar technisches Kaliumhydroxid verwendet werden kann, das bis zu 10 bis 15 Gew.-% Wasser enthält. In der alkoholischen Kaliumhydroxidlösung ist dann die entsprechende Menge Wasser vorhanden. Die Erfinder haben gefunden, dass es für Schritt (i) von Vorteil ist, wenn eine gewisse Menge an Wasser, das zum Lösen des Alkalimetallhydroxid notwendig ist, im Reaktionsgemisch vorhanden ist. Die Verwendung von trockenem Kaliummethanolat in Methanol (KM32) ist weniger bevorzugt, in diesem Falle sollte Wasser zugesetzt werden. In der erfindungsgemäßen Reaktion bevorzugt zu verwendende alkoholische Alkalimetallhydroxidlösungen sind eine etwa 15 bis 20 %-ige methanolische Kaliumhydroxidlösung oder eine etwa 10 %-ige methanolische Natriumhydroxidlösung. Im industriellen Verfahren wird bevorzugt technisches Kaliumhydroxid zum Herstellen der alkoholischen Lösung verwendet. Bevorzugt beträgt der Wassergehalt der alkoholischen Alkalimetallhydroxidlösung 0,01 bis 10 Gew.-%, besonders bevorzugt 0,05 - 5 Gew.-%, besonders bevorzugt 0,1 - 2 Gew.-%, bezogen auf das Gewicht der Lösung.

Wird Natriumhydroxid (99 %) in Alkohol (z.B. Methanol) verwendet, so enthält die Reaktionsmischung nur einen geringen Anteil an Wasser. Im Vergleich zu einer Kaliumhydroxidlösung muss dann zum Lösen der gewünschten Menge Natriumhydroxid eine größere Menge an Alkohol (z.B. Methanol) verwendet werden. Der Fachmann kann durch einfaches Experimentieren die zuzugebende Alkoholmenge herausfinden, die zum Lösen der gewünschten Menge an Natriumhydroxid oder Kaliumhydroxid notwendig ist. Diese Menge an Alkohol kann variieren.

Die alkoholische Alkalimetallhydroxidlösung wird so eingestellt, dass eine bis zu 30 Gew.-%-ige Lösung, vorzugsweise eine etwa 10 bis etwa 20 Gew.-%-ige Lösung, bezogen auf die Menge des verwendeten Alkalimetallhydroxids, vorliegt.

Nach erfolgter Umsetzung in Schritt (i) wird Malonsäureester-Mono-Alkalimetallsalz der Formel (III) ohne weitere Reinigung umgesetzt. Bevor das jedoch geschieht, werden bevorzugt zunächst der Alkohol und das ggf. im Reaktionsgemisch vorhandene Wasser aus dem Reaktionsgemisch entfernt. Das kann beispielsweise über azeotrope Destillation mit Hilfe eines Schleppmittels erfolgen. Hierzu wird bevorzugt der Reaktionsmischung nach Umsetzung ein aromatisches Lösungsmittel als Schleppmitttel zugegeben. Das Schleppmittel kann bereits von Anfang an im Reaktionsgemisch vorhanden sein oder aber erst nach Umsetzung hinzugegeben werden. Bekannte Schleppmittel sind aromatische Kohlenwasserstoffe, vor allem Toluol oder Xylol. Wird das Schleppmittel erst nach Umsetzung zugegeben, so wird bevorzugt Xylol als Schleppmittel verwendet.

Das Entfernen des in Schritt (i) ggf. vorhandenen Wassers kann selbstverständlich auch durch Verwenden eines Wasserabscheiders erfolgen. Dann kann auf das azeotrope Destillieren verzichtet werden.

Es ist vorteilhaft, bei der Destillation nach Schritt (i) nicht die gesamte Menge an aromatischer Kohlenwasserstoffverbindung zu entfernen, um eine Ablagerung von festem Malonsäureester-Alkalimetallsalz der Formel (III) im Reaktionsgefäß (Reaktor) zu vermeiden.

Da sich das Malonsäureester-Alkalimetallsalz der Formel (III), (z.B. das Malonsäureester-Natriumsalz und -Kaliumsalz) in dem aromatischen Kohlenwasserstoff nicht löst, erhält man eine Suspension von Malonsäureester-Alkalimetallsalz in dem aromatischen Kohlenwasserstoff. Diese Suspension kann ohne weiteres sofort in Schritt (ii) des erfindungsgemäßen Verfahrens eingesetzt werden. Die Ausgestaltung der Reaktion in Schritt (i) ist gegenüber den bekannten Verfahren äußerst vorteilhaft, da vorzugsweise zu keiner Zeit das Malonsäureester-Mono-Alkalimetallsalz der Formel (III) als Reinsubstanz isoliert werden muss. Eine technisch aufwendige und teure Isolierung mit anschließender Trocknung des Malonsäureester-Mono-Alkalimetallsalzes der Formel (III) kann vermieden werden.

In einer Variante [i-A] des Schrittes (i) wird die Reaktion im Schritt (i) in Substanz durchgeführt, das heißt, dass ausschließlich in dem durch die alkoholische Alkalimetallhydroxidlösung eingebrachten Alkohol, der Wasser enthalten kann, gearbeitet wird. Wenn gewünscht, kann zu der Reaktionsmischung in Schritt (i) zusätzlich noch derselbe oder ein anderer Alkohol (bevorzugt Methanol und/oder Ethanol) zugegeben werden. Die Menge des im Reaktionsgemisch vorhandenen Alkohols ist so bemessen, dass das Reaktionsgemisch während der Umsetzung in Schritt (i) gut rührbar bleibt. Aus Effizienzgründen ist bei der Umsetzung in Schritt (i-A) nur ein Alkohol zugegen, nämlich der Alkohol, der auch zur Herstellung der alkoholischen Alkalimetallhydroxidlösungen verwendet wird. Der in Variante [i-A] verwendete Alkohol kann am Reaktionsende, wie nachfolgende beschrieben, wiedergewonnen und erneut zur Herstellung der in Schritt (i) verwendeten alkoholischen Alkalimetallhydroxidlösung eingesetzt werden. Mit der Variante [i-A] kann deshalb besonders ressourcen-schonend gearbeitet werden.

In der Variante [i-A] des Schrittes (i) muss nach erfolgter Umsetzung und fast vollständigem Entfernen des im Reaktionsgemisch vorhandenen Alkohols nun das ggf. in der Reaktionsmischung vorhandene Wasser azeotrop entfernt werden. Hierzu wird dem Reaktionsgemisch eine aromatische Kohlenwasserstoffverbindung als Schleppmittel zugegeben. Solche Schleppmittel sind z.B. Toluol und Xylol, wobei in der Variante [i-A] Xylol bevorzugt verwendet wird. Xylol bildet nämlich mit dem ggf. noch vorhandenen Alkohol, insbesondere Methanol, kein Azeotrop und es kann deshalb ohne großen Aufwand recyclisiert und in Schritt (i) wiederverwendet werden.

Nach Beendigung der Umsetzung und Zugabe der aromatischen Kohlenwasserstoffverbindung, wird die Reaktionsmischung solange destilliert, bis kein Wasser mehr vorhanden ist, wobei darauf zu achten ist, dass noch eine gewisse Menge an aromatischer Kohlenwasserstoffverbindung im Reaktionsgefäß verbleibt. Der Anteil der aromatischen Kohlenwasserstoffverbindung in Bezug auf das Mono-Alkalimetallsalzes des Malonsäureesters der Formel (III) sollte vorzugsweise mindestens 50 Gew.-% betragen.

In einer anderen Variante [i-B] des Schrittes (i) ist der aromatische Kohlenwasserstoff, der anfangs als Lösungsmittel und nach Beendigung der Reaktion als Schleppmittel dient, bereits während der Umsetzung im Reaktionsgemisch vorhanden. Vorzugsweise wird in Variante [i-B] Toluol verwendet. Vorteil ist hier, dass in Schritt (i) der erforderliche Anteil an Alkohol reduziert werden kann und dass der im Reaktionsgemisch vorhandene Alkohol und das Wasser gleichzeitig (azeoptrop) abdestilliert werden.

Im erfindungsgemäßen Verfahren, kommt es zwischen Schritt (i) und (ii) zu keinem Lösungsmittelwechsel, denn die in Schritt (i) von Anfang an vorhandene oder später hinzugefügte aromatische Kohlenwasserstoffverbindung, dient in Schritt (ii) als Lösungsmittel. Gegenüber den bekannten Verfahren hat das den Vorteil, dass das Verfahren als Eintopfreaktion ausgestaltet werden kann. So können Kosten gesenkt werden und das Verfahren ist ressourcen-schonend.

Da der in Schritt (ii) zu verwendende Chloressigsäureester flüssig ist, ist es jedoch nicht zwingend notwendig neben den bereits vorhandenen aromatischen Kohlenwasserstoff noch ein weiteres Lösungsmittel in Schritt (ii) hinzuzufügen.

Die in Schritt (ii) einsetzbaren Phasentransferkatalysatoren (PTC) sind dem Fachmann bekannt. Bevorzugt wird genau ein Phasentransferkatalysator eingesetzt. Es können aber auch zwei oder noch mehr verschiedene Phasentransferkatalysatoren eingesetzt werden. Besonders geeignete und erfindungsgemäß eingesetzte Phasentransferkatalysatoren sind organische Ammonium- oder Phosphoniumsalze, insbesondere Tetraalkylammoniumsalze, Benzyltrialkylammoniumsalze, Tetraalkylphosphoniumsalze, Benzyltrialkylphosphoniumsalze sowie Gemische davon.

Im erfindungsgemäßen Verfahren werden bevorzugt organische Ammoniumsalze, insbesondere Tetraalkylammoniumsalze, Benzyltrialkylammoniumsalze verwendet. Solche Salze sind z.B. Tetra-n-butylammoniumchlorid oder -bromid, Tetra-n-butylammoniumhydrogensulfat, Tri-n-butylmethylammoniumchlorid oder -bromid, Tri-n-butylmethylammoniumhydrogensulfat, Benzyltriethylammoniumchlorid oder -bromid, Benzyltriethylammoniumhydrogensulfat, Trioktylmethylammoniumchlorid oder -bromid und Trioktylmethylammoniumhydrogensulfat.

Besonders bevorzugt wird in Schritt (ii) das kommerziell erhältliche Tetra-n-butylammoniumchlorid oder -bromid sowie das kommerziell erhältliche Trioktylmethylammoniumchlorid verwendet.

Der Phasentransferkatalysator wird in katalytischen Mengen eingesetzt und kann vom Fachmann durch Routineexperimente ermittelt werden.

Dennoch ist es von Vorteil, wenn in Schritt (ii) die Menge des verwendeten Phasentransferkatalysators im Bereich von etwa 0,01 bis etwa 30 Mol-%, bezogen auf das Monoalkalimetallsalz der Formel (III) liegt. Vorzugsweise liegt sie im Bereich von etwa 0,05 bis etwa 5, besonders bevorzugt im Bereich von etwa 0,1 bis etwa 3 Mol-%, bezogen auf das Monoalkalimetallsalz der Formel (III).

Schritt (ii) kann bei milden Reaktionsbedingungen durchgeführt werden. Dies hat den Vorteil, dass weniger Nebenkomponenten entstehen, die Reaktionsmischung einfach aufgearbeitet werden kann, was wiederum zu höheren Ausbeuten des gewünschten Zielprodukts führt.

Das Verwenden eines Phasentransferkatalysators ermöglicht, dass die erfindungsgemäße Reaktion in einem Eintopfverfahren und im Wesentlichen ohne Lösungsmittelwechsel durchgeführt werden kann.

Ein Vorteil bei der Ausgestaltung des Schrittes (ii) des erfindungsgemäßen Verfahrens ist ferner, dass der nicht umgesetzte Chloressigsäureester der Formel (IV) ohne weitere Aufarbeitung für eine erneute Reaktion mit der Verbindung der Formel (III) zur Verfügung steht, was für das kontinuierliche Arbeiten - sofern gewünscht - einen großen Vorteil darstellt.

Die in Schritt (ii) erhaltene Verbindung der Formel (V) wird ebenso in einer solchen Reinheit erhalten, dass sie ohne weitere Isolierung in Schritt (iii) eingesetzt werden kann.

Es ist jedoch bevorzugt, aus der die Verbindung der Formel (V) enthaltenden Reaktionsmischung vor Weiterreaktion in Schritt (iii) das Alkalimetallsalz zu entfernen. Dies ist mit allen Methoden nach dem Stand der Technik möglich. Bevorzugt ist es, die Reaktionsmischung einer Filtration oder einer Wasserwäsche zum Entfernen des in Schritt (ii) entstandenen Alkalimetallsalzes, insbesondere des Natriumchlorids bzw. Kaliumchlorids zu unterziehen. Sollte es notwendig sein, so kann das durch die Wasserwäsche vorhandene Wasser, wie bei Schritt (i) beschrieben, azeotrop entfernt werden.

Für die Ringschlussreaktion im Schritt (iii) kann jedes Alkalimetallalkoholat, insbesondere Natrium- oder Kaliumalkoholat, verwendet werden. Bevorzugt wird dann ein Lösungsmittel, vorzugsweise Alkohol (z.B. Methanol oder Ethanol) in Schritt (iii) zugegeben werden. Aus technischen und ökonomischen Gründen ist es bevorzugt, ein in dem entsprechenden Alkohol gelöstes Natrium- oder Kaliumalkoholat zu verwenden, das vorteilhafterweise käuflich erhältlich ist (z.B. Natriummethanolat in Methanol gelöst). Eine weitere Zugabe von Lösungsmittel kann dann entfallen.

Erfindungsgemäße Natrium- oder Kaliumalkoholate sind Natriummethanolat oder Natriumethanolat, die vorzugsweise bereits in Methanol oder Ethanol gelöst vorliegen (z.B. Natriummethanolat in Methanol gelöst oder Natriumethanolat in Ethanol gelöst).

Im Gegensatz zu dem in WO-A-2009/036899 beschriebenen Verfahren kann das erfindungsgemäße Verfahren in einem Reaktionsgefäß bzw. Reaktor durchgeführt werden. Die Zwischenverbindungen verbleiben demgemäß während der gesamten Reaktion im Reaktionsgefäß bzw. Reaktor. Es findet auch kein aufwändiger Lösungsmittelwechsel statt.

Die Umsetzungen der Schritte (i), (ii) und (iii) können bei Normaldruck (etwa 1013 mbar) durchgeführt werden. Es ist auch möglich die Reaktion im Vakuum oder bei erhöhten Druck (Überdruck) durchzuführen.

Die Umsetzungen der Schritte (i), (ii) und (iii) laufen bei geeigneter Temperatur ab, die von den verwendeten Substraten abhängt. Geeignete Temperaturen lassen sich einfach durch Routineversuche ermitteln.

Schritt (i) kann beispielsweise bei einer Temperatur im Bereich von etwa -30 °C bis etwa 50 °C, vorzugsweise von etwa 0 °C bis etwa 30 °C durchgeführt werden. Besonders bevorzugt wird die Umsetzung bei einer Temperatur im Bereich von etwa 20 °C bis etwa 30 °C durchgeführt. Bevorzugt wird Schritt (i) bei einer Temperatur im Bereich von etwa 20 °C bis etwa 30 °C und bei Normaldruck, d.h etwa 1013 mbar, durchgeführt.

Schritt (ii) kann beispielsweise bei einer Temperatur im Bereich von etwa 20 °C bis etwa 200 °C, vorzugsweise von etwa 40 °C bis etwa 150 °C durchgeführt werden. Besonders bevorzugt wird die Umsetzung bei einer Temperatur im Bereich von etwa 60 °C bis etwa 80 °C durchgeführt. Bevorzugt wird Schritt (ii) bei einer Temperatur im Bereich von etwa 60 °C bis etwa 80 °C und bei Normaldruck, d.h etwa 1013 mbar, durchgeführt.

Schritt (iii) kann beispielsweise bei einer Temperatur im Bereich von etwa 20 °C bis etwa 120 °C, vorzugsweise von etwa 20 °C bis etwa 100 °C durchgeführt werden. Besonders bevorzugt wird die Umsetzung bei einer Temperatur im Bereich von etwa 20 °C bis etwa 80 °C durchgeführt. Bevorzugt wird Schritt (iii) bei einer Temperatur im Bereich von etwa 20 °C bis etwa 80 °C und bei Normaldruck, d.h etwa 1013 mbar, durchgeführt.

Im erfindungsgemäßen Verfahren kann das molare Verhältnis der Verbindung der Formel (II) zu dem in der alkoholischen Alkalimetallhydroxidlösung verwendeten Alkalimetallhydroxid leicht variieren. Das molare Verhältnis des Alkalimetallhydroxids zum Malonsäureester sollte bevorzugt zwischen 0,5 : 1 und 1,5 : 1 sein, besonders bevorzugt zwischen 0,9 : 1 und 1,1 : 1, ganz besonders bevorzugt 1 : 1 sein. Bei Einsatz von größeren Mengen Alkalimetallhydroxid im Verhältnis zum Malonsäureester kann es verstärkt zur nicht bevorzugten Bildung eines Di-Salzes kommen. Dies führt dann auch in den Folgeschritten vermehrt zu Nebenprodukten und letztendlich zu Ausbeuteverlusten.

Dagegen kann das molare Verhältnis der Verbindung der Formel (III) zur Verbindung der Formel (IV), oder der Verbindung der Formel (V) zum Alkalimetallalkoholat der Formel (VI) breit variiert werden. Die molaren Verhältnisse der Reaktanden zueinander unterliegen im Allgemeinen keiner Beschränkung.

In Schritt (ii) ist es vorteilhaft, wenn das molare Verhältnis der Verbindung der Formel (IV) zur Verbindung der Formel (III) im Bereich von 5 bis 1, insbesondere im Bereich von 2 bis 1 liegt. Erfindungsgemäß bevorzugt liegt das molare Verhältnis im Bereich von 1 bis 1,5.

In Schritt (iii) ist es vorteilhaft, wenn das molare Verhältnis des Alkalimetallalkoholats der Formel (VI) zur Verbindung der Formel (V) im Bereich von 0,5 bis 10, insbesondere im Bereich von 1 bis 5 liegt. Erfindungsgemäß bevorzugt liegt das molare Verhältnis im Bereich von 1 bis 1,5.

Wenn nichts anderes angegeben, umfasst der Ausdruck "Alkyl" verzweigte oder unverzweigte Kohlenwasserstoffe. Erfindungsgemäße Alkylreste in der Definition der Reste R¹ bis R³ sind C₁-C₁₂-Alkyl, C₁-C₆-Alkyl, C₁-C₄-Alkyl oder C₁-C₂-Alkyl. Erfindungsgemäße Alkylreste in der Definition von Phasentransferkatalysatoren sind C₁-C₁₈-Alkyl, C₁-C₁₂- Alkyl, C₁-C₆-Alk-yl, C₁-C₄-Alkyl oder C₁-C₂-Alkyl. Beispiele solcher erfindungsgemäß zu verwendenden Alkyle sind Methyl, Ethyl, n-Propyl, isoPropyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl.

Die vorliegende Erfindung wird anhand der nachfolgenden Beispiele näher erläutert, wobei die Beispiele nicht in die Erfindung einschränkender Weise zu interpretieren sind.

### Herstellungsbeispiel 1 (erfindungsgemäß):

### Herstellung von Natrium-4-(methoxycarbonyl)-5-oxo-2,5-dihydrofuran-3-olat und Natrium-4-(ethoxycarbonyl)-5-oxo-2,5-dihydrofuran-3-olat

**Schritt (i):** 2 kg (14,83 mol, 98%) Malonsäuredimethylester werden in Substanz bei 20 °C vorgelegt und dazu werden 967,85 g (14,83 mol, 86%) KOH (gelöst in 4,89 kg Methanol) in 1 Stunde eindosiert. Es wird 1 Stunde bei 20°C nachgerührt und anschließend bei ca. 35°C Innentemperatur Methanol im Vakuum abdestilliert. Der Rückstand wird zum Entfernen von Methanol- und Wasserresten im Vakuum azeotrop mit ca. 4 kg Xylol bei 40 bis 56 °C entwässert. Die zurückbleibende Xylol-Suspension wird im nachfolgenden Schritt direkt umgesetzt.
**Schritt (ii)** Nach Zugabe von 123,7 g (0,44 mol) Tetra-n-butylammoniumchlorid und 1,95 kg (17,8 mol) Chloressigsäuremethylester zur Suspension aus Schritt (i) wird das erhaltene Reaktionsgemisch auf 60 °C erwärmt, 5 Stunden bei dieser Temperatur nachgerührt. Anschließend wird bei ca. 60°C Xylol und Chloressigsäuremthylester abdestilliert und nach Zugabe von ca. 1 kg Xylol und 3,7 kg Wasser die organische Phase bei ca. 45 °C abgetrennt und im Vakuum Xylol und Wasserreste abdestilliert.
**Schritt (iii):** Zu dem Rückstand aus Schritt (ii) wird bei 45 °C innerhalb von 1 Stunde 2,67 kg (14,84) mol) einer 30%-igen Natriummethylat-Lösung (Natriummethanolat-Lösung) in Methanol eindosiert. Die Suspension wird zunächst für 2 Stunden auf 65°C erwärmt, anschließend auf 10°C abgekühlt, 1 Stunde bei dieser Temperatur verrührt und filtriert. Der Rückstand wird mit 594 g Methanol als Verdrängungswäsche gewaschen und im Vakuum getrocknet. Man erhält 2,159 kg (97,51% HPLC-Reinheit) Natrium-4-(methoxycarbonyl)-5-oxo-2,5-dihydrofuran-3-olat. Bezogen auf eingesetztes Malonsäuredimethylester entspricht das einer isolierten Ausbeute von 79%.

### ¹H-NMR (D₂O, 298K) δ: 3,73 s (3H), 4,42 s (2H).

### Herstellungsbeispiele 2-12:

### Schritt (ii):

Gekauftes 11,95 g (75,7 mmol) Kaliummethylmalonat werden in 71,25 g verschiedener Lösungsmittel/Lösungsmittelgemische gelöst/suspendiert, wobei hinsichtlich Temperaturen und Katalysatorzusatz gemäß Tabelle 1 variiert wurde.

Anschließend werden 8,23g (75,0 mol) Chloressigsäuremethylester zugesetzt und die erhaltenen Mischungen bei Raumtemperatur gerührt.

**Tabelle 1 (Experimente 2-6 nicht erfindungsgemäß, Experimente 7 - 12 erfindungsgemäß)**

| **Experiment** | **LösungsmittelGemisch)** | **Zusatz** | **Temperatur** | **Umsatz (GC)** |
|---|---|---|---|---|
| 2 | DMF | - | RT | > 98% |
| 3 | Cyclohexan | - | RT | - |
| 4 | Toluol | - | RT | - |
| 5 | Toluol: DMF = 9:1 | - | RT | - |
| 6 | Toluol | PEG 500 | RT | - |
| 7 | Toluol | 0,18 mol-% TBAB + PEG 500 | RT | 93% |
| 8 | Toluol | 0,18 mol-% TBAB | RT | 93% |
| 9 | Toluol | 0,08 mol-% TBAB | RT | 76% |
| 10 | Toluol | 0,18 mol-% TBAB | 50°C | > 98% |
| 11 | Toluol | 0,12 mol-% TBAB | 50°C | > 95% |
| 12 | ½ Toluol | 0,12 mol-% TBAB | 50°C | > 98% |

| | | | | |
|---|---|---|---|---|
| TBAB: Tetra-n-butylammoniumbromid (Phasentransferkatalysator) PEG 500: Polyethylenglykol mit einer mittleren Molekülmasse von 500 g/mol | | | | |

Ergebnis: Aus den experimentellen Ergebnissen der Herstellungsbeispiele 2 bis 12 ist erkennbar, dass
1. es möglich ist, das polare DMF durch das unpolare Toluol zu ersetzen, wobei der Zusatz eines Phasentransferkatalysators (hier TBAB) erforderlich ist. (s. Exp. 2 - 8),
2. die untere Grenze der Phasentransferkatalysator-Zugabe bevorzugt bei ∼ 0,1 mol-% liegt, und
3. eine Temperaturerhöhung vorteilhaft ist.
4. Vergleich mit dem Verfahren gemäß dem Stand der Technik (WO-A-2009/036899): Beispiele 2 und 4 sind gemäß Verfahren nach WO-A-2009/036899 durchgeführt. Beispiel 2 mit dem polaren Lösemittel DMF und Beispiel 4 mit dem unpolaren Lösemittel Toluol. Der Vergleich der Beispiel 4 (gemäß WO-A-2009/036899) und Beispiel 8 (erfindungsgemäß) zeigt, dass bei Umstellung von polarem auf unpolares Lösemittel zwingend ein Phasentransferkatalysator erforderlich ist, da sonst keine Umsetzung zum Produkt erfolgt. Der Vergleich von Beispiel 2 (gemäß WO-A-2009/036899) mit polarem Lösemittel und Beispiel 8 (erfindungsgemäß) zeigt, dass bei der Reaktion im erfindungsgemäßen zweiphasigen Verfahren vergleichbare Umsätze erzielt werden können wie in dem Verfahren gemäß WO-A-2009/036899. Aufgrund der Zweiphasigkeit im erfindungsgemäßen Verfahren ist die Abtrennung und Isolierung des Produkts aber wesentlich einfacher durchzuführen als in dem einphasigen Verfahren gemäß WO-A-2009/036899 unter Verwendung des polaren Lösemittels.

### Herstellungsbeispiel 13 (mit Toluol) (erfindungsgemäß):

Schritt (i): Zunächst werden 4150 g Methanol in einem Reaktor vorgelegt und unter kräftigem Rühren und unter Kühlung nach und nach 1858 g (29 mol; 86%) festes Kaliumhydroxid zugesetzt, um eine 30 %-ige Lösung von KOH in Methanol zu erhalten. Diese wird zu einer Lösung von 3839 g (29 mol) Dimethylmalonat in 5700 g Toluol vorgelegt. Und unter kräftigem Rühren über 1 h hinweg zugegeben. Anschließend wird das Reaktionsgemisch für 2 - 4 h bei Raumtemperatur nachgerührt. Dann werden weitre 5700 g Toluol zugesetzt und solange Methanol abdestilliert bis kein Methanol mehr im Filtrat des Sumpfes erkennbar ist. (GC-Flächengehalt < 0,1%).
Schritt (ii): Zu der toluolischen Kaliummethylmalonat-Suspension aus Schritt (i) werden 96 g (0,3 mol) Tetrabutylammoniumbromid (TBAB) zugesetzt und auf 65 °C erhitzt. Dann werden 2655 g (24,5 mol) Chloressigsäuremethylester zugegeben und das Reaktionsgemisch für mindestens 6h bei 65 °C gerührt. Nach vollständigem Umsatz wird die Mischung abgekühlt und mit 6540 g Wasser extrahiert. Die toluolische Phase wird destilliert, so dass etwa die Hälfte des Toluols abdestilliert und das Wasser entfernt wird.
Schritt (iii): Die toluolische Lösung wird auf 60 °C gebracht und mit 5010 g NM30-Lösung (Natriummethanolat) versetzt. Nach beendeter Zugabe wird die Reaktionsmischung weitere 4 h bei ca. 60 °C gerührt. Das Reaktionsgemisch wird für mindestens 2 h auf ≤ 5 °C abgekühlt. Das ausgefallene Produkt wird abfiltriert und zweimal mit je 1500 g Methanol gewaschen und getrocknet.

### Ausbeute: 74,6 % (Reinheit (HPLC): 94,5 %)

### Herstellungbeispiel 14 (mit Xylol) (erfindungsgemäß):

Die Synthese erfolgt analog zu der in Herstellungsbeispiel 13, wobei anstelle von Toluol Xylol eingesetzt wird.

Ausbeute: 57,7 % (Reinheit (HPLC): 95,8%).

## Patentansprüche

1. Verfahren zur Herstellung eines Alkalimetallsalzes eines 4-Hydroxy-2-oxo-2,5-dihydrofuran-3 carbonsäureesters der Formel (I) 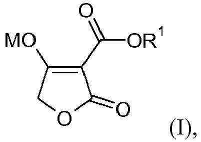 das die folgenden Schritte umfasst:
Schritt (i): Umsetzen eines Malonsäureesters der allgemeinen Formel (II) 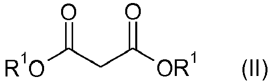 mit einer alkoholischen Alkalimetallhydroxidlösung zum entsprechenden Malonsäureester-Mono-Alkalimetallsalz der Formel (III) 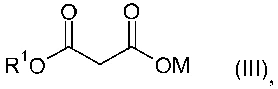
Schritt (ii): Umsetzen des Malonsäureester-Mono-Alkalimetallsalzes der Formel (III) aus Schritt (i) mit einem Chloressigsäureester der Formel (IV) 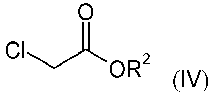 in Gegenwart mindestens eines Phasentransferkatalysators zu einer Verbindung der Formel (V) 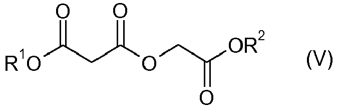 wobei der Phasentransferkatalysator ein organisches Ammonium- oder Phosphoniumsalz ist,
Schritt (iii): Umsetzen der Verbindung der Formel (V) durch Zugabe eines Alkalimetallalkoholats der allgemeinen Formel (VI)
M'OR³ (VI)
in einer Ringschlussreaktion wodurch das Alkalimetallsalz des 4-Hydroxy-2-oxo-2,5-dihydrofuran-3-carbonsäureesters der Formel (I) hergestellt wird,
worin in den vorgenannten allgemeinen Formeln (I) bis (VI)
R¹, R² und R³ jeweils unabhängig voneinander für C₁-C₁₂-Alkyl stehen;
M und M' unabhängig voneinander für ein Alkalimetall in der entsprechenden Oxidationsstufe steht.

2. Verfahren nach Anspruch 1, bei dem Schritt (i) in Anwesenheit eines aromatischen Kohlenwasserstoffs durchgeführt wird oder worin dem Reaktionsgemisch während oder nach der Umsetzung in Schritt (i) ein aromatischer Kohlenwasserstoff zugegeben wird.

3. Verfahren nach Anspruch 2 oder 3, bei dem die Umsetzung in Schritt (i) in Anwesenheit von Wasser erfolgt.

4. Verfahren nach einem der Ansprüche 1 bis 3, bei dem nach erfolgter Umsetzung in Schritt (i) aus dem Reaktionsgemisch Alkohol und ggf. Wasser zumindest teilweise entfernt werden, wobei das Malonsäureester-Mono-Alkalimetallsalz der Formel (III) dann als Suspension in dem aromatischen Kohlenwasserstoff vorliegt.

5. Verfahren nach einem der Ansprüche 1 bis 4, worin
R¹, R² und R³ jeweils für Methyl oder Ethyl steht; und
M und M' jeweils für Natrium oder Kalium in der entsprechenden Oxidationsstufe steht.

6. Verfahren nach einem der Ansprüche 1 bis 5, worin als alkoholische Alkalimetallhydroxidlösung in Schritt (i) eine methanolische oder ethanolische Kaliumhydroxidlösung bzw methanolische oder ethanolische Natriumhydroxidlösung verwendet wird, die, bezogen auf die Menge des verwendeten Natrium- oder Kaliumhydroxids, als eine bis zu 30 Gew.-%-ige Lösung verwendet wird.

7. Verfahren nach einem der Ansprüche 1 bis 6, worin der aromatische Kohlenwasserstoff aus Schritt (i) Xylol oder Toluol ist und das im Reaktionsgemisch vorhandene Wasser und Alkohol aus dem Reaktionsgemisch destillativ entfernt wird.

8. Verfahren nach Anspruch 7, worin das Lösungsmittel Toluol ist und der im Reaktionsgemisch vorhandene Alkohol und das vorhandene Wasser durch azeotrope Destillation entfernt werden.

9. Verfahren nach Anspruch 7, worin in Schritt (i) das in der Reaktionsmischung vorhandene Wasser durch Zugabe von Xylol und anschließende azeoptrope Destillation entfernt wird, wobei aber zuerst der in der Reaktionsmischung vorhandene Alkohol destillativ entfernt wird.

## Claims

1. Method for preparing an alkali metal salt of a 4-hydroxy-2-oxo-2,5-dihydrofuran-3-carboxylic ester of the formula (I) 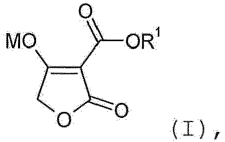 comprising the following steps:
step (i): reacting a malonic ester of the general formula (II) 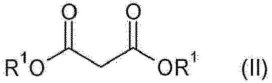 with an alcoholic alkali metal hydroxide solution to give the corresponding malonic ester mono-alkali metal salt of the formula (III) 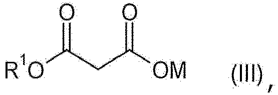
step (ii): reacting the malonic ester monoalkali metal salt of the formula (III) from step (i) with a chloroacetic ester of the formula (IV) 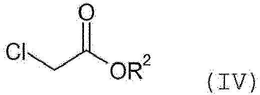 in the presence of at least one phase transfer catalyst to give a compound of the formula (V) 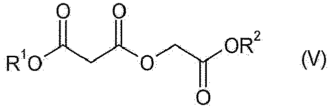 wherein the phase transfer catalyst is an organic ammonium or phosphonium salt,
step (iii): reacting the compound of the formula (V) by addition of an alkali metal alkoxide of the general formula (VI)
M'OR³ (VI)
in a ring closure reaction as a result of which the alkali metal salt of 4-hydroxy-2-oxo-2,5-dihydrofuran-3-carboxylic ester of the formula (I) is produced, where in the above-mentioned general formulae (I) to (VI)
R¹, R² and R³ are each independently C₁-C₁₂-alkyl;
M and M' are each independently an alkali metal in the corresponding oxidation state.

2. Method according to Claim 1, in which step (i) is carried out in the presence of an aromatic hydrocarbon or in which an aromatic hydrocarbon is added to the reaction mixture during or after the reaction in step (i).

3. Method according to Claim 2 or 3, in which the reaction in step (i) is conducted in the presence of water.

4. Method according to any of Claims 1 to 3, in which alcohol and optionally water are at least partially removed from the reaction mixture on completion of the reaction in step (i), wherein the malonic ester monoalkali metal salt of the formula (III) is then present as a suspension in the aromatic hydrocarbon.

5. Method according to any of Claims 1 to 4, where
R¹, R² and R³ are in each case methyl or ethyl; and
M and M' are in each case sodium or potassium in the corresponding oxidation state.

6. Method according to any of Claims 1 to 5, wherein the alcoholic alkali metal hydroxide solution used in step (i) is a methanolic or ethanolic potassium hydroxide solution or methanolic or ethanolic sodium hydroxide solution, which, based on the amount of sodium hydroxide or potassium hydroxide used, is used as a solution up to 30% by weight.

7. Method according to any of Claims 1 to 6, wherein the aromatic hydrocarbon from step (i) is xylene or toluene and the water and alcohol present in the reaction mixture are removed from the reaction mixture by distillation.

8. Method according to Claim 7, wherein the solvent is toluene and the alcohol present in the reaction mixture and the water present are removed by azeotropic distillation.

9. Method according to Claim 7, wherein the water present in the reaction mixture in step (i) is removed by addition of xylene and subsequent azeotropic distillation, but wherein the alcohol present in the reaction mixture is initially removed by distillation.

## Revendications

1. Procédé pour la préparation d'un sel de métal alcalin d'un ester de l'acide 4-hydroxy-2-oxo-2,5-dihydrofuranne-3-carboxylique de formule (I) 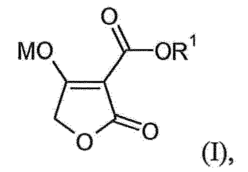 comprenant les étapes suivantes, consistant à :
étape (i) : transformer un ester de l'acide malonique de formule générale (II) 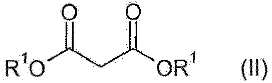 avec une solution alcoolique d'un métal alcalin en mono-sel de métal alcalin de l'ester de l'acide malonique correspondant de formule (III) 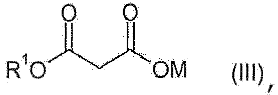
étape (ii) : transformer le mono-sel de métal alcalin de l'ester de l'acide malonique de formule (III) de l'étape (i) avec un ester de l'acide chloroacétique de formule (IV) 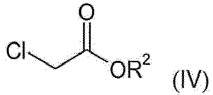 en présence d'au moins un catalyseur de transfert de phase en composé de formule générale (V) 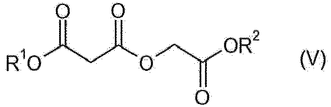 le catalyseur de transfert de phase étant un sel organique d'ammonium ou de phosphonium,
étape (iii) : transformer le composé de formule (V) par addition d'un alcoolate de métal alcalin de formule générale (VI)
M'OR³ (VI)
dans une réaction de fermeture de cycle, suite à quoi le sel de métal alcalin de l'ester de l'acide 4-hydroxy-2-oxo-2,5-dihydrofuranne-3-carboxylique de formule (I) est préparé, où, dans les formules générales susmentionnées (I) à (VI)
R¹, R² et R³ représentent à chaque fois, indépendamment les uns des autres, C₁-C₁₂-alkyle ;
M et M' représentent, indépendamment l'un de l'autre, un métal alcalin dans l'étage d'oxydation correspondant.

2. Procédé selon la revendication 1, dans lequel l'étape (i) est réalisée en présence d'un hydrocarbure aromatique ou dans lequel le mélange réactionnel est additionné, pendant ou après la transformation dans l'étape (i), d'un hydrocarbure aromatique.

3. Procédé selon la revendication 2 ou 3, dans lequel la transformation dans l'étape (i) a lieu en présence d'eau.

4. Procédé selon l'une quelconque des revendications 1 à 3, dans lequel, après la transformation dans l'étape (i), on élimine l'alcool et le cas échéant l'eau, au moins partiellement, du mélange réactionnel, le mono-sel de métal alcalin de l'ester de l'acide malonique de formule (III) se trouvant alors sous forme de suspension dans l'hydrocarbure aromatique.

5. Procédé selon l'une quelconque des revendications 1 à 4, dans lequel
R¹, R² et R³ représentent à chaque fois méthyle ou éthyle ; et
M et M' représentent à chaque fois sodium ou potassium dans l'étage d'oxydation correspondant.

6. Procédé selon l'une quelconque des revendications 1 à 5, dans lequel on utilise, comme solution alcoolique d'hydroxyde de métal alcalin dans l'étape (i), une solution méthanolique ou éthanolique d'hydroxyde de potassium ou une solution méthanolique ou éthanolique d'hydroxyde de sodium qui, par rapport à la quantité d'hydroxyde de sodium ou de potassium utilisé, est utilisée sous forme d'une solution de jusqu'à 30% en poids.

7. Procédé selon l'une quelconque des revendications 1 à 6, dans lequel l'hydrocarbure aromatique de l'étape (i) est le xylène ou le toluène et l'eau et l'alcool présents dans le mélange réactionnel sont éliminés par distillation du mélange réactionnel.

8. Procédé selon la revendication 7, dans lequel le solvant est de toluène et l'alcool et l'eau présents dans le mélange réactionnel sont éliminés par distillation azéotrope.

9. Procédé selon la revendication 7, dans lequel, dans l'étape (i), l'eau présente dans le mélange réactionnel est éliminée par addition de xylène et distillation azéotrope consécutive, l'alcool présent dans le mélange réactionnel étant cependant d'abord éliminé par distillation.
